(19) Europäisches Patentamt
European Patent Office
Office européen des brevets

(11) Veröffentlichungsnummer: **0 214 528**
**A2**

(12) # EUROPÄISCHE PATENTANMELDUNG

(21) Anmeldenummer: 86111580.6

(22) Anmeldetag: 21.08.86

(51) Int. Cl.⁴: C07D 401/06, A61K 31/55, C07B 57/00

(30) Priorität: 05.09.85 DE 3531682

(43) Veröffentlichungstag der Anmeldung: 18.03.87 Patentblatt 87/12

(84) Benannte Vertragsstaaten: AT BE CH DE FR GB IT LI LU NL SE

(71) Anmelder: **Dr. Karl Thomae GmbH**
**Postfach 1755**
**D-7950 Biberach (Riss)(DE)**

(72) Erfinder: **Trummlitz, Günter, Dr. Dipl.-Chem.**
**Buchenweg 27**
**D-7951 Warthausen(DE)**
Erfinder: **Engel, Wolfhard, Dr. Dipl.-Chem.**
**Mozartstrasse 13**
**D-7950 Biberach 1(DE)**
Erfinder: **Eberlein, Wolfgang, Dr. Dipl.-Chem.**
**Obere Au 6**
**D-7950 Biberach 1(DE)**
Erfinder: **Mihm, Gerhard, Dr. Dipl.-Chem.**
**Nickeleshalde 5/1**
**D-7950 Biberach 1(DE)**
Erfinder: **Giachetti, Antonio, Prof. Dr.**
**Via Guerrini Olindo 3**
**Mailand(IT)**

(54) **(+)-6-Chlor-5,10-dihydro-5-[(1-methyl-4-piperidinyl)acetyl]-11H-dibenzo[b,e][1,4]diazepin-11-on, seine Isolierung und Verwendung als Arzneimittel.**

(57) Beschrieben wird das (+)-6-Chlor-5,l0-dihydro-5-[(l-methyl-4-piperidinyl)-acetyl]-llH-dibenzo[b,e]-[l,4]-diazepin-ll-on, seine Isolierung aus einem Enantiomerengemisch und seine Verwendung als Arzneimittel.

Die Verbindung zeichnet sich durch eine starke Wirkung gegen Ulcera des Magen-Darm-Traktes aus.

EP 0 214 528 A2

## ( + )-6-Chlor-5,l0-dihydro-5-[(l-methyl-4-piperidinyl)acetyl]-llH-dibenzo[b,e][l,4]diazepin-ll-on, seine Isolierung und Verwendung als Arzneimittel

Die Erfindung betrifft ( + )-6-Chlor-5,l0-dihydro-5-[(l-methyl-4-piperidinyl)acetyl]-llH-dibenzo[b,e]-[l,4]diazepin-ll-on, seine Isolierung aus einem Enantiomerengemisch und seine Verwendung als Arzneimittel.

In der Europäischen Patentanmeldung Nr. 83 l00 677.0 (Veröffentlichungs-Nr. 0 085 892) sind substituierte Dibenzodiazepinone, Verfahren zu ihrer Herstellung und sie enthaltende Arzneimittel beschrieben. Beschrieben wurde unter anderem auch die Verbindung 6-Chlor-5,l0-dihydro-5-[(l-methyl-4-piperidinyl)acetyl]-llH-dibenzo[b,e][l,4]diazepin-ll-on der Formel I:

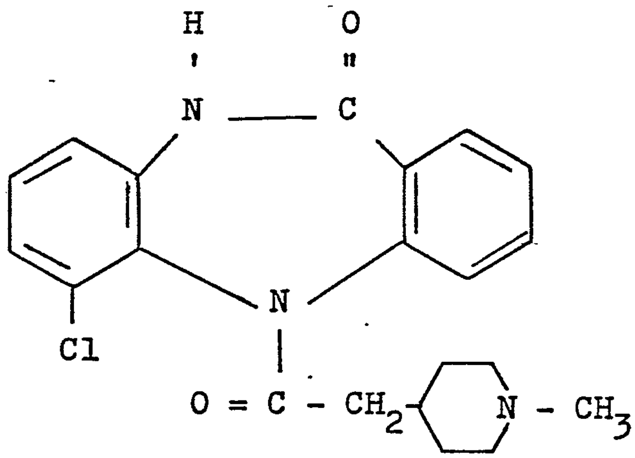

(I)

Es wurde nun völlig überraschend gefunden, daß die Verbindung der Formel I in bei Raumtemperatur stabilen, enantiomeren Formen vorliegt und daß das Enantiomerengemisch trennbar ist. Diese Verhaltensweise war aus den folgenden Gründen nicht vorhersehbar:

Bei Betrachtung der Formel I ist kein asymmetrisches Atom erkennbar. Es ist jedoch bekannt, daß der Siebenring bei derartigen Verbindungen nicht planar, sondern in einer Bootform vorliegt und somit die Tricyclen vom Typ der Formel I prinzipiell in zwei zueinander spiegelbildlichen chiralen Konformationen auftreten können. Beide Formen stehen jedoch in einem Gleichgewicht und sollten nicht trennbar sein. So konnte für die Substanz Pirenzepin der Formel II

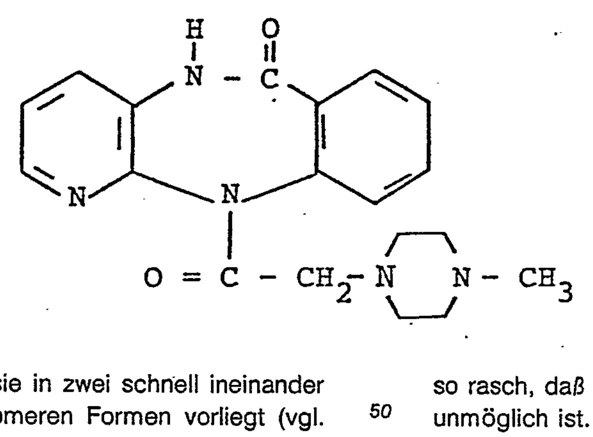

(II)

gezeigt werden, daß sie in zwei schnell ineinander übergehenden enantiomeren Formen vorliegt (vgl. Arzneim.-Forsch./Drug Res. 34(II), 8, 849-859 - (l984)). Die Umwandlung von der einen in die andere Form erfolgt aber bereits bei Raumtemperatur so rasch, daß eine Auftrennung in die Einzelformen unmöglich ist. Ein entsprechendes Verhalten mußte auch für die Verbindung der Formel I angenommen werden.

Jetzt wurde überraschend gefunden, daß die Verbindung der Formel I sich in ihre optischen Antipoden auftrennen läßt und, was noch mehr überrascht, daß die optischen Antipoden selbst bei Temperaturen bis 150°C nicht racemisieren. Diese Tatsachen erlauben die Anwendung nur eines Enantiomeren, in diesem Fall der physiologisch stark wirksamen (+)-Form, in der Therapie der Erkrankungen des Magen-Darm-Traktes.

Für die Verbindung der Formel I sind die folgenden enantiomeren Konformationen des Tricyclus möglich:

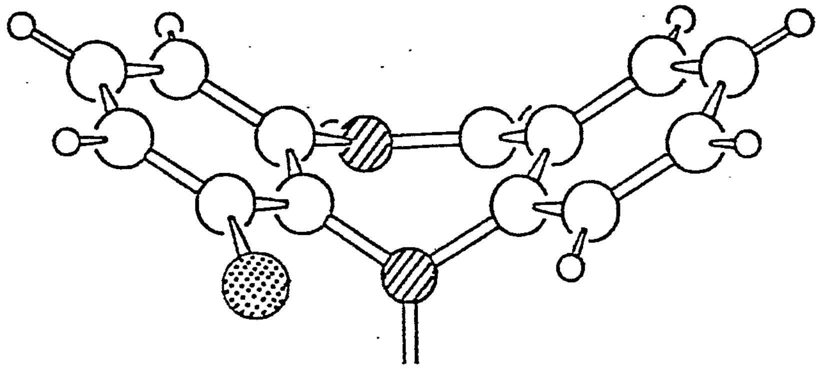

Ia

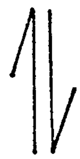

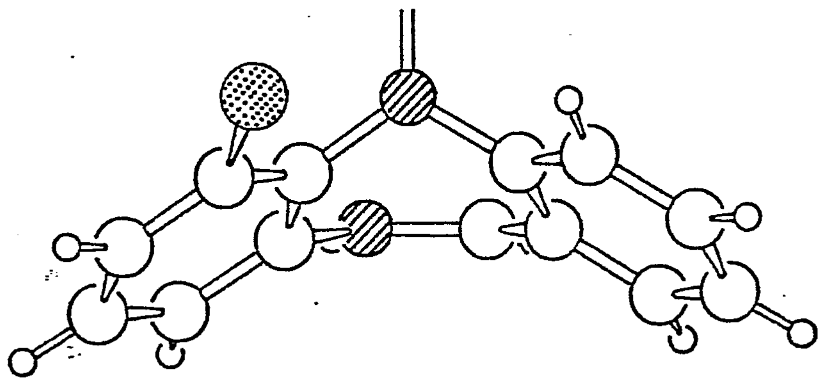

Ib

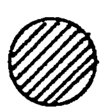 = Stickstoffatom

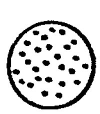 = Chloratom

Es wurde gefunden, daß das Enantiomere mit dem spezifischen optischen Drehwert von $[\alpha]^{20}_{D}$ = + 165,9° (c = 0,7l; in Chloroform) die biologisch wirksame Form darstellt und in seinem tricyclischen Molekülteil die Konformation Ia hat, was durch Röntgenstrukturanalyse nachgewiesen wurde. Hierzu wurden mit L-Weinsäure und den Enantiomeren der Formel I Salzkristalle hergestellt und röntgengenographisch untersucht; die Auswertung der Meßergebnisse ergab die Konformation Ia.

Die Auftrennung eines nach den in der eingangs genannten Europäischen Patentschrift Nr. 83 100 677.0 erwähnten Verfahren erhaltenen Racemats in die optisch aktiven Antipoden läßt sich nach an sich bekannten Verfahren durchführen, beispielsweise unter Verwendung einer optisch aktiven Säure. Als optisch aktive Säuren kommen vor allem die L-(+)-oder D-(-)-Weinsäure, eines ihrer Derivate, wie die (+) oder (-)-Diacetylweinsäure, (+) oder (-)-Monomethyltartrat oder die (+)-Camphersäure in Betracht. Bevorzugt wird zur Auftrennung die L-(+)-oder D-(-)-Weinsäure verwendet. Das Gemisch der Enantiomeren der Formel I wird mit einer der vorstehend beschriebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis von 50:50, verwendet. Durch Zugabe von Alkali zu den jeweiligen Salzen können die enantiomeren Basen freigesetzt werden.

Das so erhaltene (+)-6-Chlor-5,10-dihydro-5-[(1-methyl-4-piperidinyl)-acetyl]-11H-dibenzo[b,e][1,4]-diazepin-11-on ist, im Gegensatz zu der (-)-Form, stark antiulcerogen und magensäuresekretionshemmend wirksam, es zeigt zudem einen günstigen Effekt auf verschiedene andere Erkrankungen des Magen-Darm-Traktes, unter denen Colon irritabile besonders hervorgehoben sei. Es hat sich gezeigt, daß man zur Erzielung der gleichen Effekte von der (+)-Form nur ungefähr die Hälfte der Dosis benötigt, die bei Verwendung des Enantiomerengemisches appliziert werden müßte; von der (-)-Form benötigt man dagegen mindestens die 50-fache Menge zur Erzielung der gleichen Effekte.

Es wurde die Magenschutzwirkung der beiden Enantiomeren der Formel I an Hand eines Modellversuches untersucht; die Methodik und die Ergebnisse werden wie folgt beschrieben.

Wirkung auf Ratten nach Pylorusligatur:

Methode: Verwendet wurden während 24 Stunden nüchtern gehaltene männliche Wistar-Ratten (Körpergewicht zwischen 100 und 125 g), die freien Zugang zu Wasser hatten. Die Ratten wurden in Gruppen von jeweils 10 Tieren aufgeteilt. Unter Ethernarkose wurde der Bauchraum durch einen Längsschnitt geöffnet und der Pylorus abgebunden. Vor der Pylorusligatur wurden die in Kochsalzlösung aufgelösten Substanzen in dosierter Form intravenös (0,1 ml/100 g Körpergewicht) verabreicht, die intravenöse Applikation erfolgte unmittelbar vor der Pylorusligatur; Kontrolltiere erhielten nur die Kochsalzlösung. Zwei Stunden nach der Ligatur wurden die Tiere getötet und der Magen vorsichtig entnommen. Die gesammelten Mageninhalte wurden 15 Minuten bei 4000 Umdrehungen pro Minute zentrifugiert, das Überstehende wurde zur Bestimmung des Magensaftvolumens abgetrennt. Mittels eines pH-Meßgerätes (I.T.T. Radiometer PHM 62) wurde in 1 ml-Proben des Magensaftes die Azidität durch Titration auf pH 7 bestimmt.

Die Gesamtsäure wurde aus dem Produkt aus gemessenem Volumen und Azidität (µEq.ml) berechnet und als µEq über 2 oder 4 Stunden ausgedrückt. Die ermittelten Daten der einzelnen, vorbehandelten Ratten wurden jeweils mit den von Kontrollgruppen erhaltenen Durchschnittswerten verglichen und als prozentuale Abweichung ausgedrückt. Die prozentualen Werte wurden in Probits transformiert und einer linearen Regressionsanalyse unterworfen, um die $ED_{50}$-Werte für Volumen und Gesamtsäuresekretion unter Beachtung einer 95%igen Vertrauensgrenze zu bestimmen.

Ergebnisse:

| Substanz | ED$_{50}$-Wert mg/kg Ratte i.v. |
|---|---|
| (+)-Form | 0,18 |
| (-)-Form | >> 3* |

* Bei 3 mg/kg liegt erst eine 1,6%ige Abweichung von den Durchschnittswerten unbehandelter Kontrollgruppen vor.

Aus dem Versuch ergibt sich eindeutig, daß die (+)-Form der Träger der Wirkung ist.

Das rechtsdrehende Enantiomer der Formel I kann nach Umsetzung mit anorganischen oder organischen Säuren auch in Form anderer physiologisch verträglicher Salze vorliegen. Als Säuren haben sich hierzu beispielsweise Salzsäure, Bromwasserstoffsäure, Schwefelsäure, (-)-Weinsäure, Fumarsäure oder Zitronensäure als geeignet erwiesen.

Ein weiterer Gegenstand der Erfindung sind Arzneimittel, die das rechtsdrehende Enantiomer der Formel I enthalten. Die (+)-Form läßt sich hierzu in an sich bekannter Weise in die üblichen pharmazeutischen Zubereitungsformen, z.B. in Lösungen, Suppositorien, Tabletten, Dragees, Kapseln oder Teezubereitungen einarbeiten. Die Tagesdosis liegt im allgemeinen zwischen 0,001 und 0,5 vorzugsweise 0,002 und 0,25, insbesondere 0,005 und 0,1 mg/kg Körpergewicht, die gegebenenfalls in Form mehrerer, vorzugsweise 1 bis 3 Einzelgaben, zur Erzielung des gewünschten Effektes verabreicht wird.

Die folgenden Beispiele dienen zur näheren Erläuterung der Erfindung:

Beispiel 1

(+)-6-Chlor-5,10-dihydro-5-[(1-methyl-4-piperidinyl)-acetyl]-11H-dibenzo[b,e][1,4]diazepin-11-on-L-(+)-tartrat

25 g L-(+)-Weinsäure ("natürliche" Weinsäure) werden in 100 ml Ethanol gelöst und in eine zur Siedehitze erwärmte Lösung von 127,9 g (±)-6-Chlor-5,10-dihydro-5-[(1-methyl-4-piperidinyl)-acetyl]-11H-dibenzo[b,e][1,4]diazepin-11-on in 900 ml Ethanol eingetragen.

Aus dieser Lösung kristallisierten beim Abkühlen 68,5 g 6-Chlor-5,10-dihydro-5-[(1-methyl-4-piperidinyl)-acetyl]-11H-dibenzo[b,e][1,4]diazepin-11-on-L-(+)-tartrat aus. Es handelt sich hierbei um eine 90:10 Anreicherung der (+)-Form. Die Mutterlauge wurde zur Isolierung der (-)-Form (siehe unten) verwendet.

50 g des obigen Tartrates (angereicherte (+)-Form) wurden in 100 ml Wasser gelöst, mit 200 ml Methylenchlorid und, unter Rühren, mit festem Kaliumcarbonat solange versetzt, bis die wäßrige Phase sich auf dem Kolbenboden absetzte. Die abgetrennte wäßrige Phase wurde noch zweimal mit Methylenchlorid extrahiert. Die vereinten organischen Phasen wurden über Magnesiumsulfat getrocknet und anschließend im Vakuum eingeengt. Der Rückstand (44,1 g) wurde mit 8,6 g L-(+)-Weinsäure in 500 ml heißem Ethanol umgesetzt. Beim Abkühlen kristallisierten 42,1 g (+)-6-Chlor-5,10-dihydro-5-[(1-methyl-4-piperidinyl)acetyl]-11H-dibenzo[b,e][1,4]diazepin-11-on-L-(+)-tartrat aus.

Durch die Aufarbeitung der Mutterlaugen, insbesondere nach Abtrennung der angereicherten (-)-Form mittels Kristallisation mit D-(-)-Weinsäure, ist es möglich, weitere 28,25 g der (+)-Form zu isolieren.

Die Ausbeute an reinem (+)-6-Chlor-5,10-dihydro-5-[(1-methyl-4-piperidinyl)-acetyl]-11H-dibenzo[b,e][1,4]diazepin-11-on-L-(+)-tartrat bezogen auf racemisches 6-Chlor-5,10-dihydro-5-[(1-methyl-4-piperidinyl)-acetyl]-11H-dibenzo[b,e][1,4]-diazepin-11-on liegt somit bei 85% der Theorie.

Schmelzpunkt: 175°C (Zersetzung).

$C_{21}H_{22}ClN_3O_2 \times 3/4 C_4H_6O_6$ (496,45)

| | | | | | | | | |
|---|---|---|---|---|---|---|---|---|
| Ber.: | C | 58,07 | H | 5,38 | Cl | 7,14 | N | 8,46 |
| Gef.: | | 57,88 | | 5,60 | | 6,88 | | 8,32 |

Beispiel 2

(+)-6-Chlor-5,l0-dihydro-5-[(l-methyl-4-piperidinyl)-acetyl]-llH-dibenzo[b,e][l,4]diazepin-ll-on

5 g (+)-6-Chlor-5,l0-dihydro-5-[(l-methyl-4-piperidinyl)-acetyl]-llH-dibenzo[b,e][l,4]diazepin-ll-on-L-(+)-tartrat wurden in Wasser gelöst, mit Pottasche versetzt und mit Methylenchlorid extrahiert. Die Extrakte wurden eingeengt und mit Ethanol mehrmals nachgedampft. Man erhält 3,9 g (95 % der Theorie) (+)-6-Chlor-5,l0-dihydro-5-[(l-methyl-4-piperidinyl)acetyl]-llH-dibenzo[b,e][l,4]-diazepin-ll-on.

Schmelzpunkt: ll2°C; $[\alpha]_D^{20}$ = + 165,9° - (c = 0,7l; $CHCl_3$).

Die optische Reinheit des so erhaltenen Produktes (alle anderen wurden ebenso charakterisiert) wurde neben der Konstanz der optischen Drehung bei weiteren Umkristallisationen durch chirale HPLC überprüft. Man verwendete eine Säule von J.T. Baker Chemical Co.: Bakerbond 250 × 4,6 mm (R)-N-3,5-Dinitrobenzoyl-leucin auf 5 µm Aminopropyl-Silica, Säule mit Rheodym Einlaßventil Nr. 7l25 mit 250 µl Probeschleife (Spectra Physics Integrator System I) in Verbindung mit einem Perkin-Elmer Liquid Chromatographen - (Series IIIB), Spectrophotometer LC 75 und Scheiber Typ 56l; als mobile Phase wurde n-Hexan/Ethanol/Methanol (200:8:8) verwendet.

Es konnte so hachgewiesen werden, daß der Gehalt an (+)-Form bei über 98 % liegt und das andere Enantiomer unter 2 % vorkommt.

Beispiel 3

(-)-6-Chlor-5,l0-dihydro-5-[(l-methyl-4-piperidinyl)-acetyl]-llH-dibenzo[b,e][l,4]diazepin-ll-on-D-(-)-tartrat

Die zum Beispiel l analoge Umsetzung des Enantiomerengemisches mit D-(-)-Weinsäure liefert das (-)-Enantiomere in Form seines D-(-)-Tartrates.

Schmelzpunkt: l75°C (Zersetzung).

$C_{21}H_{22}ClN_3O_2$ × $3/4C_4H_6O_6$ (496,45)

| | | | | | | | | |
|---|---|---|---|---|---|---|---|---|
| Ber.: | C | 58,07 | H | 5,38 | Cl | 7,14 | N | 8,46 |
| Gef.: | | 57,82 | | 5,49 | | 6,90 | | 8,25 |

Beispiel 4

(-)-6-Chlor-5,l0-dihydro-5-[(l-methyl-4-piperidinyl)-acetyl]-llH-dibenzo[b,e][l,4]diazepin-ll-on

Aus (-)-6-Chlor-5,l0-dihydro-5-[(l-methyl-4-piperidinyl)acetyl]-llH-dibenzo[b,e][l,4]diazepin-ll-on-D-(-)-tartrat wurde analog Beispiel 2 die Base freigesetzt.

Schmelzpunkt: ll2°C; $[\alpha]_D^{20}$ = -165,6° (c = 0,79; Chloroform).

Beispiel 5

(-)-6-Chlor-5,l0-dihydro-5-[(l-methyl-4-piperidinyl)-acetyl]-llH-dibenzo[b,e][l,4]diazepin-ll-on-D-(-)-tartrat

Aus der Mutterlauge des Beispiel l, die in einem ca. 75:25 angereicherten Mol-Verhältnis das (-)-Enantiomere enthält, läßt sich durch jeweiliges Freisetzen mit wäßriger Pottaschelösung und zweimaliges Umkristallisieren unter Zusatz von D-(-)-Weinsäure aus heißem Ethanol das (-)-6-Chlor-5,l0-dihydro-5-[(l-methyl-4-piperidinyl)-acetyl]-llH-dibenzo[b,e][l,4]diazepin-ll-on-D-(-)-tartrat in 82%iger Ausbeute gewinnen.

Schmelzpunkt: l75°C (Zersetzung),

$C_{21}H_{22}ClN_3O_2$ × $3/4C_4H_6O_6$ (496,45)

| | | | | | | | | |
|---|---|---|---|---|---|---|---|---|
| Ber.: | C | 58,07 | H | 5,38 | Cl | 7,14 | N | 8,46 |
| Gef.: | | 58,02 | | 5,19 | | 7,02 | | 8,32 |

Beispiel 6

(+)-6-Chlor-5,l0-dihydro-5-[(l-methyl-4-piperidinyl)-acetyl]-llH-dibenzo[b,e][l,4]diazepin-ll-on-L-(+)-tartrat

Aus der Mutterlauge des Beispiels 3, die in einem ca. 75:25 angereicherten Verhältnis das (+)-Entiomere enthält, läßt sich durch Freisetzen und Kristallisieren in Gegenwart von L-(+)-Weinsäure aus heißem Ethanol das (+)-6-Chlor-5,l0-dihydro-5-[(l-methyl-4-piperidinyl)-acetyl]-llH-dibenzo[b,e][l,4]-diazepin-ll-on-L-(+)-tartrat in 86%iger Ausbeute gewinnen.

Schmelzpunkt: l75°C (Zersetzung),

$C_{21}H_{22}ClN_3O_2 \times 3/4C_4H_6O_6$ (496,45).

| | | | | | | | | |
|---|---|---|---|---|---|---|---|---|
| Ber.: | C | 58,07 | H | 5,38 | Cl | 7,14 | N | 8,46 |
| Gef.: | | 57,91 | | 5,12 | | 6,80 | | 8,09 |

Beispiel 7

Racemisierungsversuche

In unterschiedlichen pH-Bereichen (pH l-9), in unterschiedlichen Lösungsmitteln (Wasser, Glykol) und in Form ihrer Schmelze konnte für das (+)-6-Chlor-5,l0-dihydro-5-[(l-methyl-4-piperidinyl)-acetyl]-llH-dibenzo[b,e][l,4]diazepin-ll-on bei Temperaturen bis l50°C über eine Zeitdauer von l5 Minuten keine Racemisierung der Substanz des Beispiels l nachgewiesen werden. Der Drehwert blieb konstant, durch Anwendung der HPLC konnte kein entstandenes (-)-Enantiomeres nachgewiesen werden.

Im folgenden wird die Herstellung pharmazeutischer Anwendungsformen anhand einiger Beispiele beschrieben:

Beispiel I

Tabletten mit 0,5 mg (+)-6-Chlor-5,l0-dihydro-5-[(l-methyl-4-piperidinyl)acetyl]-llH-dibenzo[b,e][l,4]-diazepin-ll-on

Zusammensetzung:

1 Tablette enthält:

| | |
|---|---|
| Wirkstoff | 0,5 mg |
| Milchzucker | 152,5 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

Tablettengewicht: 220 mg

Stempel: 9mm

Beispiel II

Dragées mit 0,5 mg (+)-6-Chlor-5,10-dihydro-5-[(1-methyl-4-piperidinyl)acetyl]-11H-dibenzo[b,e][1,4]-diazepin-11-on

Die nach Beispiel I hergestellten Tabletten werden nach be kanntem Verfahrem mit einer Hülle überzogen, die im wesentlichen aus Zucker und Talkum besteht. Die fertigen Dragées werden mit Hilfe von Bienenwachs poliert.

Dragéegewicht: 300 mg

Beispiel III

Ampullen mit 0,2 mg (+)-6-Chlor-5,10-dihydro-5-[(1-methyl-4-piperidinyl)acetyl]-11H-dibenzo[b,e][1,4]-diazepin-11-on-hydrochlorid

Zusammensetzung:

1 Ampulle enthält:

Wirkstoff 0,2 mg

Natriumchlorid 8,0 mg

Des. Wasser ad 1 ml

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1ml-Ampullen abgefüllt.

Sterilisation: 20 Minuten bei 120°C.

Beispiel IV

Suppositorien mit 1 mg (+)-6-Chlor-5,10-dihydro-5-[(1-methyl-4-piperidinyl)acetyl]-11H-dibenzo[b,e][1,4]-diazepin-11-on

Zusammensetzung:

1 Zäpfchen enthält:

| | |
|---|---|
| Wirkstoff | 1,0 mg |
| Zäpfchenmasse (z.B. Witepsol W 45 R) | 1699,0 mg |
| | 1700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.

Zäpfchengewicht: 1,7 g

Beispiel V

Tropfen mit (+)-6-Chlor-5,10-dihydro-5-[(1-methyl-4-piperidinyl)acetyl]-11H-dibenzo[b,e][1,4]-diazepin-11-on

Zusammensetzung:

100 ml Tropflösung enthalten:

p-Hydroxybenzoesäuremethylester 0,035 g

p-Hydroxybenzoesäurepropylester 0,015 g

Anisöl 0,05 g

Menthol 0,06 g

Ethanol rein I0,0 g

Wirkstoff 0,I g

Natriumcyclamat I,0 g

Glycerin I5,0 g

Dest. Wasser ad I00,0 ml

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf I00 ml aufgefüllt und schwebeteilchenfrei filtriert.

**Ansprüche**

I.) (+)-6-Chlor-5,I0-dihydro-5-[(I-methyl-4-piperidinyl)-acetyl]-IIH-dibenzo[b,e][I,4]-diazepin-II-on und dessen physiologisch verträgliche Salze mit anorganischen oder organischen Säuren.

2.) Arzneimittel, enthaltend (+)-6-Chlor-5,I0-dihydro-5-[(I-methyl-4-piperidinyl)-acetyl]-IIH-dibenzo[b,e][I,4]-diazepin-6-on oder eines seiner physiologisch verträglichen Salze mit anorganischen oder organischen Säuren.

3.) Verwendung der Verbindung gemäß Anspruch I als ulcus-oder magensäuresekretionshemmendes Mittel.

4.) Verfahren zur Herstellung eines Arzneimittels gemäß Anspruch 2, dadurch gekennzeichnet, daß eine Verbindung gemäß Anspruch I auf nicht chemischem Wege in einen oder mehrere inerte Trägerstoffe und/oder Verdünnungsmittel eingearbeitet wird.

5.) Verfahren zur Isolierung von (+)-6-Chlor-5,I0-dihydro-5-[(I-methyl-4-piperidinyl)-acetyl]-IIH-dibenzo[b,e][I,4]-diazepin-II-on und, gegebenenfalls, zur Herstellung seiner Salze mit anorganischen oder organischen Säuren, dadurch gekennzeichnet, daß man das Racemat der Verbindung der Formel I

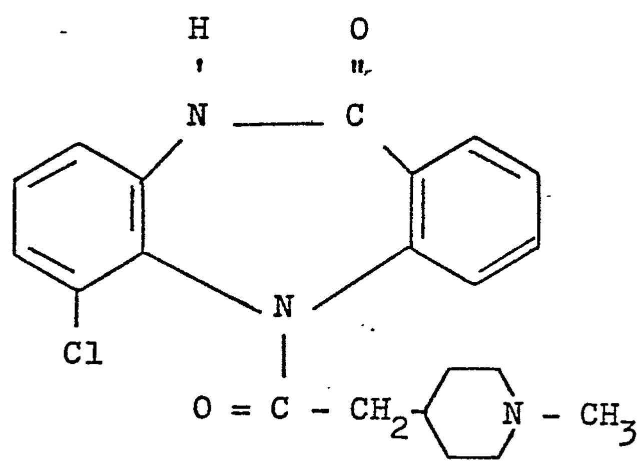

(I)

mit einer optisch aktiven Säure in einem Lösungsmittel in diastereomere Salze überführt, diese unter Ausnutzung ihrer unterschiedlichen Löslichkeiten trennt und aus dem so erhaltenen Salz der (+)-Form durch Zugabe von Alkali die freie Base isoliert und, gegebenenfalls die freie Base anschließend mittels anorganischer oder organischer Säuren in entsprechende Salze überführt.

6.) Verfahren gemäß Anspruch 5, gekennzeichnet durch die Verwendung von L-(+)-und D-(-)-Weinsäure als organische Säuren und von Ethanol als Lösungsmittel.